# EUROPEAN PATENT APPLICATION

(11) **EP 3 395 235 A1**
(43) Date of publication of application: **31.10.2018**
(21) Application number: 16878957.6
(22) Date of filing: 22.12.2016
(51) Int. Cl.: A61B 5/00, A61B 1/04

(54) **MEDICAL SYSTEM**

(30) Priority: 25.12.2015 JP 2015253138
(71) Applicant: Westunitis Co., Ltd., Kita-ku Osaka-shi, Osaka 530-0011 (JP)
(72) Inventor: FUKUDA, Takahito, Osaka-shi, Osaka 530-0011 (JP); KINOSHITA, Tadatoshi, Osaka-shi, Osaka 530-0011 (JP)
(74) Representative: Ström & Gulliksson AB
(86) International application number: PCT/JP2016/088468
(87) International publication number: WO 2017/111071

(57) **Abstract**

To provide a medical system that is easy to confirm a display screen and easy to switch an image. A medical camera 2 is connected to a first input terminal 14a of a switching device 6. A medical image output device 4 for outputting a medical image is connected to a second input terminal 14b. An HMD 10 mounted on a face of a user is connected to a first output terminal 16a. An external monitor 12 is connected to a second output terminal 16b. Initially, a medical image from the medical image output device 4 is output and displayed on the HMD 10. A camera image from the medical camera 2 is output and displayed on the external monitor 12. When a control switch 66 is operated, images displayed on the HMD 10 and the external monitor 12 may be switched.

## Description

### TECHNICAL FIELD

The present invention relates to a medical system for displaying medical images such as images of a diseased site during operation, MRIs or the like.

### BACKGROUND ART

A medical system is used to display images of a diseased site imaged during operation or medical images. Because these systems are used during operation, the systems are demanded to have excellent operability.

In a system disclosed in, for example, JP-A-2009-183686, an operation screen of medical equipment such as an endoscope and an operation screen of non-medical instruments such as a room camera and a liquid crystal display device are commonalized and made easy to operate.

Further, in medical cares or the like, a camera imaging a surgical part or the like is demanded to have a small size and excellent operability. As a camera in a medical field, an endoscope (JP-A-1994-167658) is used. However, the endoscope has a limited intended purpose and has not been used in ordinary operations.

Further, an intraoral camera for dental use is proposed (JP-A- 2008-86554). In place of a conventional dental mirror, an inside of the mouth is imaged and displayed as an image.

### SUMMARY OF INVENTION

### TECHNICAL PROBLEM

Although the operability is improved by commonalization of operation screens in Patent Document 1, since various kinds of information is shown on many displays, it is not easy to check the information during medical operation.

Unfortunately, in the medical cameras or intraoral cameras of Patent Document 2 or Patent Document 3, a user such as a medical staff or a dental surgeon cannot readily set a camera to a site to be imaged during treatment or operation and cannot readily check the displayed image. In particular, the operability when used as a wearable system is not well considered.

The present invention intends to solve any of the problems described above and to provide a medical system that is easy for the operator to check a display screen and readily switch an image, further to provide a camera and a wearable system having excellent operability.

### SOLUTION TO PROBLEM

Some features of the present invention that may be independently applied will be listed.
(1) A medical system according to the present invention includes: a medical camera that capture an image and outputs the camera image; a medical image output device that outputs a medical image; a head-mounted display mounted on a user; an external monitor that the user or a person other than the user may see; and a switching device having a selection control part, to which the camera image from the medical camera and medical data from a medical data output device are input, and which selects at least one of the camera image and the medical data to be shown in each of the head-mounted display and the external monitor..
   Accordingly, a medical system that is excellent in the visibility by the user and that is readily checked by staff other than the user is provided.
(2) The switching device according to the present invention includes: a first input terminal for inputting a camera image from a medical camera that takes an image and outputs the camera image; a second input terminal for inputting a medical image from a medical image output device that outputs the medical image; a first output terminal for outputting an image signal to a head-mounted display mounted on a user; a second output terminal for outputting the image signal to an external monitor that a user or a person other than the user can see; and a selection control part, to which the camera image from the first input terminal and the medical image from the second input terminal are input, and which selects at least one of the camera image or the medical image to be shown in each of the first output terminal and the second output terminal.
   Therefore, a medical system that is excellent in the visibility by the user, and persons other than the user can also easily confirm may be provided.
(3) In the switching device according to the present invention, the selection control part changes which image to be shown according to a signal from a control switch.
   Accordingly, a switching operation can be performed by operating a control switch.
(4) In the medical system according to the present invention, the selection control part switches an image that is output to the first output terminal when an on-signal from the control switch is continuously input for a predetermined time period or shorter and switches an image that is output to the second output terminal when the on-signal from the control switch is continuously input exceeding the predetermined time period.
   Therefore, single switch can control outputs to two output terminals.
(5) In the medical system according to the present invention, the control switch is a foot switch.
   Therefore, since the control switch is operated by a foot, an operation due to a hand is not disturbed.
(6) In the medical system according to the present invention, the selection control part at least flips vertically, rotates by 90, or mirrors an image, which is output to the first output terminal or the second output terminal or to both upon receiving a control signal.
   Therefore, while switching an image to be displayed, a display state of the image can be also switched.
(7) In the medical system according to the present invention, the selection control part includes recording means for recording an image from the first input terminal or the second input terminal or both thereof on a portable recording medium.
   Therefore, while switching an image to be displayed, an image may be recorded.
(8) In the medical system according to the present invention, a medical camera is mounted on a body of the user.
   Therefore, an image may be taken following a movement of the user and the image may be displayed.
(9) In the medical system according to the present invention, the medical camera is an intraoral camera.

Therefore, a dental surgeon can use it for dental treatment.

The "medical camera" is a camera used in medical cares, and a camera for capturing the image of surgical body parts, surgical rooms, patient rooms, and the insides of a mouth, an abdomen, or an internal organ. The medical camera is not only cameras mounted wearable on human body, but also cameras installed stationary.
(a) The flexible camera according to the present invention includes: a base part; a flexible arm one end of which is attached to the base part; and an apex part that is attached to the other end of the flexible arm and provided with a camera and a light source.
   Therefore, directions of the camera and the light source may be readily changed.
(b) The wearable system according to the present invention is a wearable system that includes a flexible camera mounted on eyeglasses and a head-mounted display that is mounted on the eyeglasses and is connected to the flexible camera, the flexible camera including the base part, the flexible arm one end of which is attached to the base part, and the apex part that is attached to the other end of the flexible arm and is provided with the camera and the light source.
   Therefore, it can be provided a wearable system having a camera that can readily change the direction of the camera and the direction of the light source.
(c) In the wearable system according to the present invention, the head-mounted display includes: the base part; the flexible arm one end of which is attached to the base part; and the apex part that is attached to the other end of the flexible arm and is provided with the display.
   Therefore, it can be provided the wearable system that can readily adjust a position of the display.
(d) In the wearable system according to the present invention, the flexible arm includes a coil obtained by winding a wire as a hollow and a covering tube that contacts with an outer periphery of the coil, is elongated such that a contraction force is generated in a direction in which the adjacent wires are pressure-welded, or is formed such that an inner diameter in a natural state is smaller than an outer diameter of the coil, and is elongated such that a contraction force is generated in a radial direction.
   Therefore, a wearable system having a camera or a display that is easy to hold a bent position while being easily bent can be obtained.
(e) The intraoral camera according to the present invention is an intraoral camera that includes a handle part and an imaging part that is formed at a predetermined angle at an apex of the handle part. The imaging part includes a camera and a light source, the imaging part is formed such that a center part is high and a periphery part is low. The center part is provided with an imaging hole for the camera to take an image, and the periphery part is provided with an illumination hole for taking out illumination light by allowing light from the light source to pass through.
   Therefore, a camera that can appropriately take the image of the inside of an oral cavity may be obtained.
(f) The wearable system according to the present invention is a wearable system that includes an intraoral camera mounted on eyeglasses and the head-mounted display that is mounted on the eyeglasses and connected to the intraoral camera. The intraoral camera includes a handle part and an imaging part that is formed at a predetermined angle at an apex of the handle part. The imaging part includes a camera and a light source. The imaging part is formed such that a center part is high and a periphery part is low. The center part is provided with an imaging hole for the camera to take an image, and the periphery part is provided with an illumination hole for taking out illumination light by allowing light from the light source to pass through.
   Therefore, the wearable system having a camera that can appropriately take an image of the inside of the oral cavity can be provided.
(g) In the wearable system according to the present invention, the head-mounted display includes a base part, a flexible arm one end of which is attached to the base part, and an apex part that is attached to the other end of the flexible arm and is provided with a display.

Therefore, the wearable system, by which a position of the display can be readily adjusted, can be provided.

### BRIEF DESCRIPTION OF THE DRAWINGS

FIG. 1 is an entire configuration of a medical system according to one embodiment of the present invention.
FIG. 2 is an example of a surgery that uses a medical system.
FIG. 3 is an example of a wearable device 602 that an operator 600 wears.
FIG. 4 is an example of a mounting part 20.
FIGs. 5A and 5B show details of a guide member 15 and an attachment guide 30.
FIG. 6 is an example of a medical camera 2.
FIG. 7 is a chassis structure of the medical camera 2.
FIG. 8 is a structure of a flexible arm 9.
FIG. 9a is an outline view of a tube 90, and FIG.9b is a cross-sectional view of the tube 90.
FIGs. 10a, 10b and 10c are diagrams showing a procedure for covering a coil 80 with the tube 90.
FIG. 11 is a diagram showing a circuit of the medical camera 2.
FIG. 12 is an example of an HMD 10.
FIG. 13 is a diagram showing a circuit of a switching device 6.

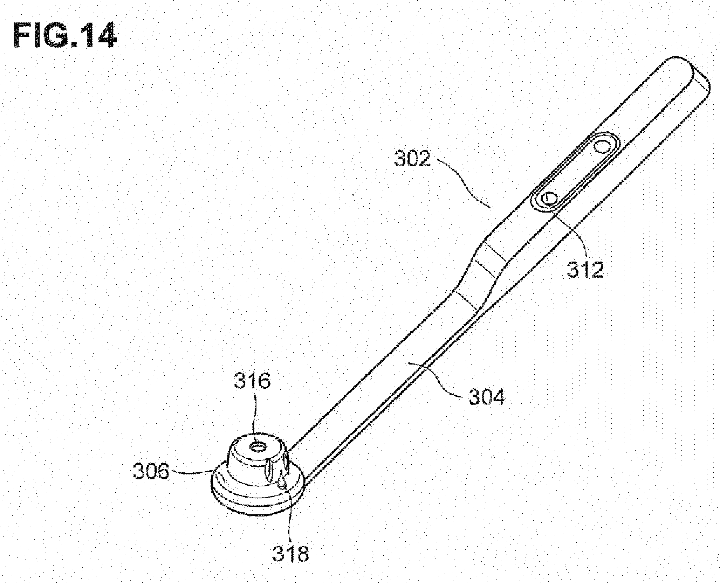

FIG. 14 is an example of an intraoral camera 302.

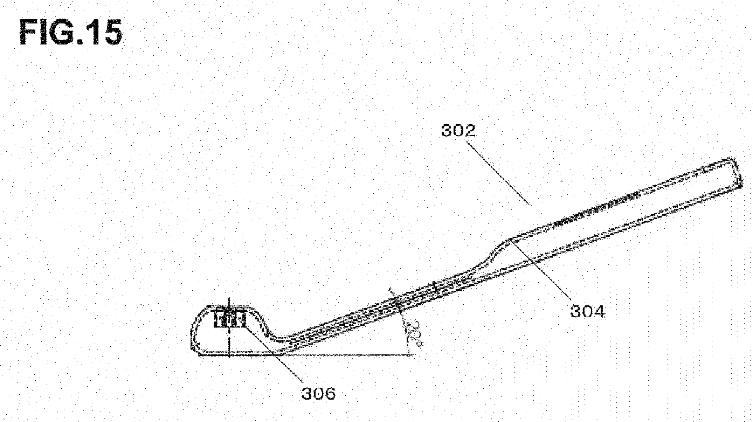

FIG. 15 is across-sectional view of the intraoral camera 302.
FIG. 16A, FIG. 16B and FIG. 16C are diagrams showing a micro-SD card 800 for recording image data and an SD card adapter 802.
FIG. 17 is a circuit block diagram of the SD card adapter 802.

### EMBODIMENT OF THE INVENTION

### 1. Entire Configuration

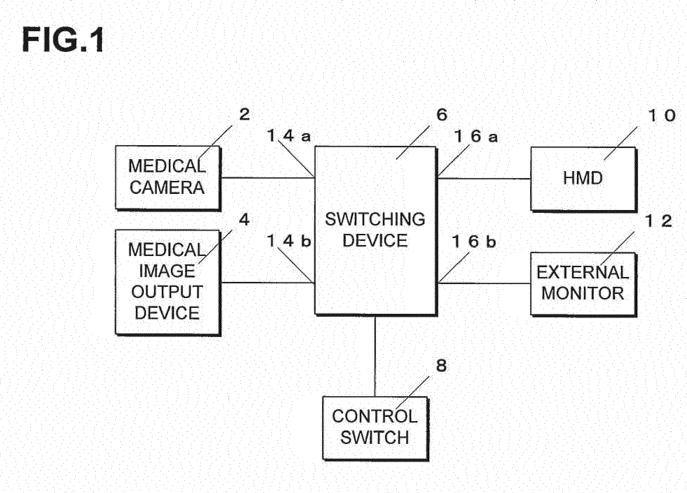

In FIG. 1, an entire configuration of a medical system according to one embodiment of the present invention is shown. To a first input terminal 14a of a switching device 6, a medical camera 2 is connected. This medical camera 2 is mounted on, for example, a face of a medical staff to take the image of a surgical part. To a second input terminal 14b, a medical image output device 4 for outputting medical images is connected. These medical images of the patient are for example MRI, SPECT, or X-ray images captured in advance or vital data (electrocardiogram or the like) acquired real time during a surgery.

To a first output terminal 16a of the switching device 6, a head-mounted display (HMD) 10 mounted on a face of a user is connected. To a second output terminal 16b, an external monitor 12 is connected.

For example, initially, a medical image from the medical image output device 4 is output and displayed on the HMD 10. On the external monitor 12, the camera image of the medical camera 2 is displayed.

Thus, a medical staff who is performing an operation can check the present vital data and the like of the patient by the HMD 10 with a slight sight line movement. Further, staff in the surroundings of the surgeon who is performing an operation may check a surgical site by seeing the external monitor 12. In particular, the external monitor 12 is effective in such a case when there are many staff in the surroundings and the surgical site cannot be seen directly. Further, since the surgical site may be seen with the same sight line as the medical staff, the external monitor 12 may be used for guidance.

Upon receiving a signal from a control switch 8, the switching device 6 switches an image displayed on the HMD 10 and the external monitor 12. In the present embodiment, when the control switch 8 is kept in an on-state for a predetermined time period or longer (for example, 0.5 second or longer), an image given to the second output terminal 16b (input of the external monitor 12) is switched from the medical camera 2 to the medical image output device 4. Further, when the control switch 8 is kept in the on-state for a predetermined time or longer, an image given to the second output terminal 16b (input of the external monitor 12) is once more switched from the medical image output device 4 to the medical camera 2. Thus, by keeping the control switch 8 in the on-state for a predetermined time period or longer, an image given to the second output terminal 16b is alternately switched to the medical camera 2 or the medical image output device 4.

When the control switch 8 is kept in the on-state for a time shorter than a predetermined time period (for example, shorter than 0.5 second), an image given to the first output terminal 16a is alternately switched to the medical camera 2 or the medical image output device 4.

Therefore, the medical staff can switch images to be displayed on the HMD 10 and the external monitor 12 only by operating the control switch 8.

### 2. Hardware Configuration and Operation

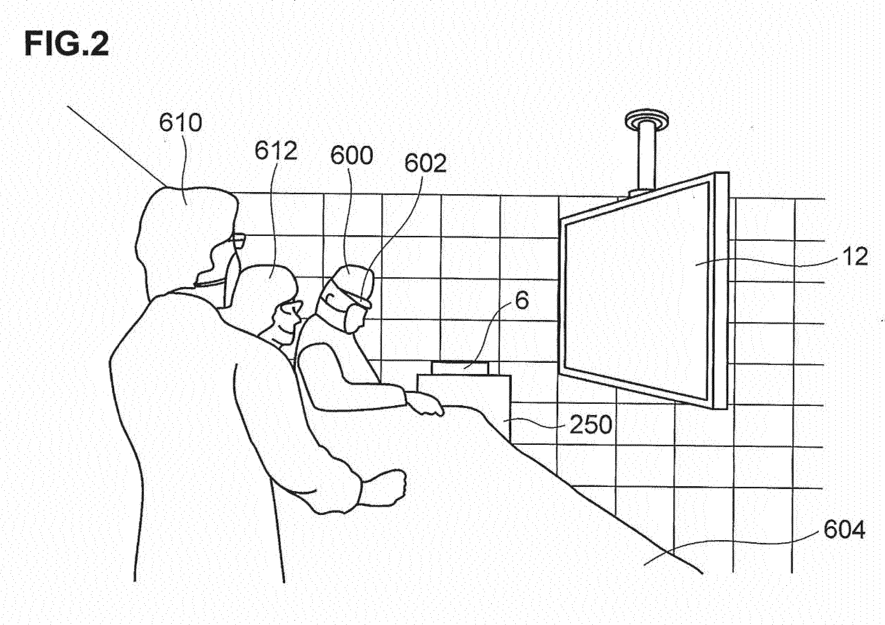

In FIG. 2, a situation of an operation that uses the above medical system is shown. In a surgical room, a surgical table 604 is provided. Further, the external monitor 12 is installed. An operator 600 wears a wearable device 602 on a face. Other than the operator 600, there are staff 610 and 612 in the surroundings of the surgical table 604.

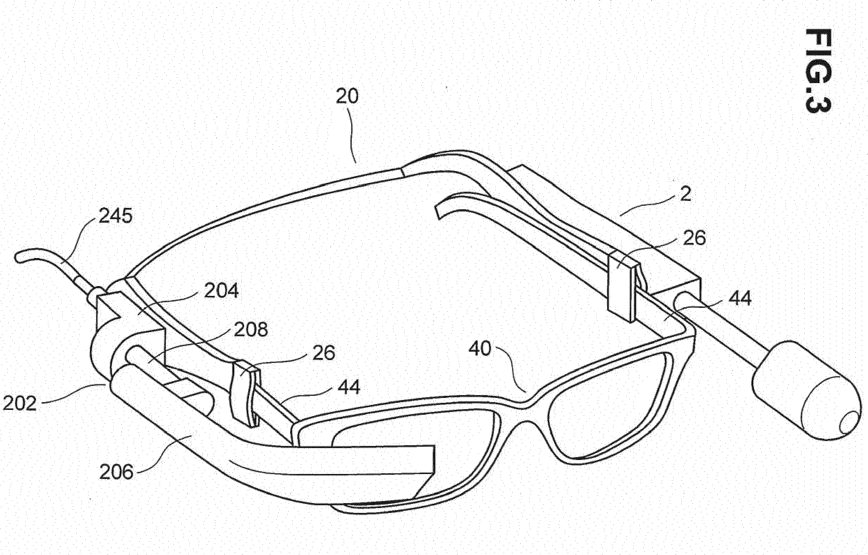

In FIG. 3, the wearable device 602 that the operator 600 wears is shown. A mounting fixture 20 is attached to the eyeglasses 40. To this mounting fixture 20, the HMD 10 and the medical camera 2 are mounted.

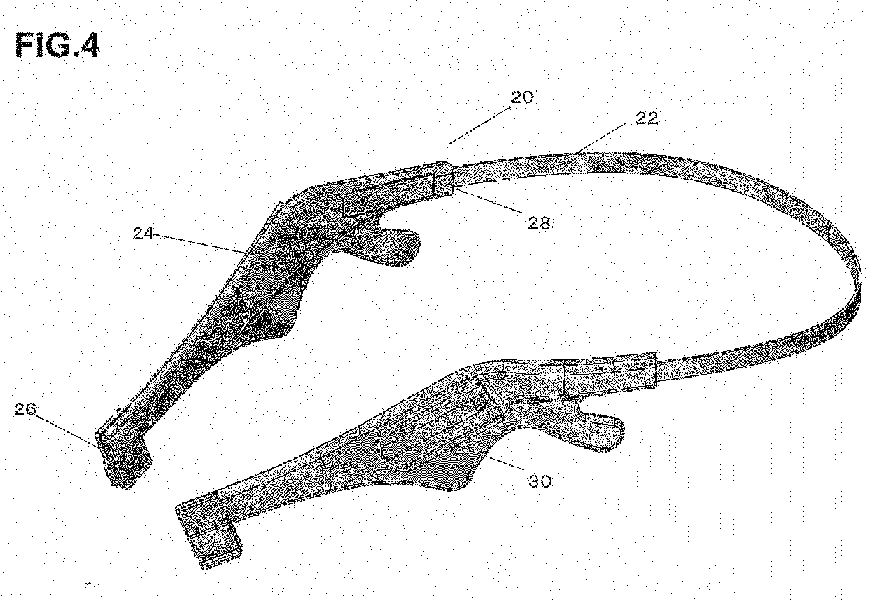

A detail of the mounting fixture 20 is shown in FIG. 4. The mounting fixture 20 includes a metallic U-shaped band 22 and a retention member 24 made of a synthetic resin and attached to an apex of the U-shaped band 22. To a back end of the retention member 24, a bonding part 28 is provided, and the apex of the U-shaped band 22 is slidably inserted. By sliding the U-shaped band 22 such that the U-shaped band 22 comes into contact with a back side of a neck of a wearer, a solid mounting state is realized. At the apex of the retention member 24, a holding part 26 formed into a U-shape opened on a lower side is formed. Further, an attachment guide 30 is formed on a side surface of the retention member 24.

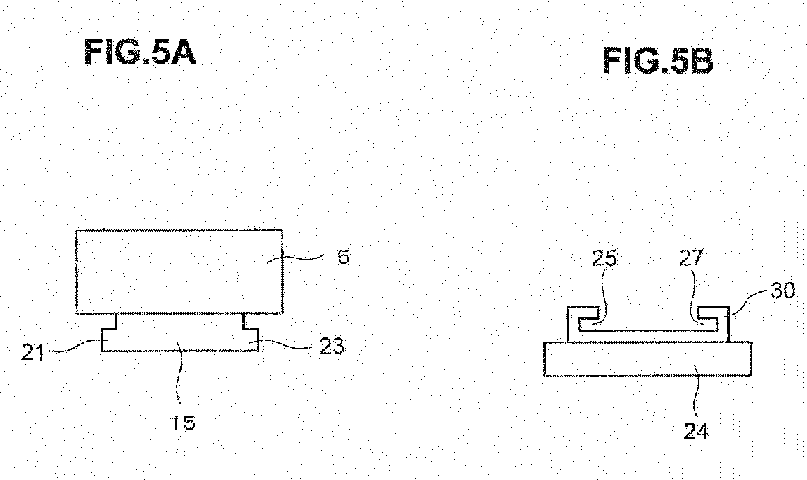

A cross-section of the attachment guide 30 is shown in FIG. 5B. Concave parts 25 and 27 are provided on both sides. On the other hand, the base part 5 of the medical camera 2 is provided with a guide member 14 such as shown in FIG. 5A (provided on a back side of a lower body 4a of FIG. 2). When convex parts 21 and 23 of the guide member 15 of the medical camera 2 are engaged and slid to the concave parts 25 and 27 of the attachment guide 30 of the retention member 24 of FIG. 5B, the medical camera 2 may be fixed to the mounting fixture 20.

As shown in FIG. 3, to a temple 44 of the eyeglasses 40, the holding part 26 of the mounting fixture 20 is tucked from an upper part, and the mounting fixture 20 is fixed to the eyeglasses 40. Further, to the attachment guide 30 of the mounting fixture 20, the guide member 14 of the medical camera 2 is slid and inserted and fixed. Thus, by attaching the mounting fixture 20 to the temple 44 of the eyeglasses 40, the medical camera 2 is fixed.

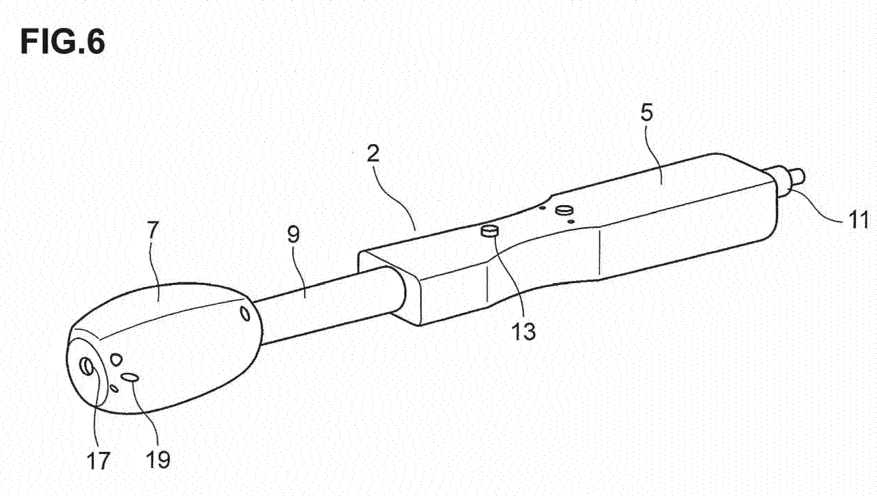

In FIG. 6, an external appearance of the medical camera 2 according to one embodiment of the present invention is shown. The base part 5 and the apex part 7 are provided. The base part 5 and the apex part 7 are connected by a flexible arm 9. In the apex part 7, a camera and a light source are housed. An imaging hole 17 is provided at the apex part 7 for taking an image by this camera. Further, for illumination by the light source, the apex part 7 is provided with an illumination hole 19.

The camera is provided with a plurality (6 in the present embodiment) of light sources (LEDs) corresponding to each of the illumination holes 19 corresponding to the imaging hole 17. In the present embodiment, a position of an apex of the illumination hole 19 is formed lower than a position of an apex of the imaging hole 17 (nearer to the base part 5). Thus, without the light from the light source directly coming into the camera, an appropriate imaging operation may be performed.

The base part 5 is provided with an illuminance changing switch 13. Every time the switch 13 is pushed, an output of the light source is switched from a lower to a higher in multiple steps. After reaching a highest output, the output returns to a lowest output.

To a back end part of the base part 5, an HDMI (trade mark) cable 11 is connected. This HDMI (trade mark) cable 11 is connected to the first input terminal 14a of the switching device 6.

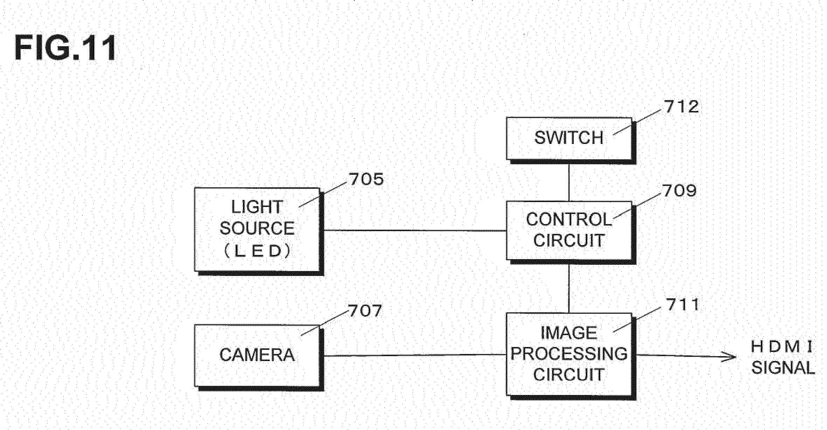

In FIG. 11, a circuit configuration of the medical camera 2 is shown. In the present embodiment, a light source (LED) 705 and a camera 707 are housed in the apex part 7. A control circuit 709 and an image processing circuit 711 are housed in the base part 5.

When a switch 712 is pushed, the control circuit 709 controls the power given to the light source 705 in order to change a light amount as described above. The image processing circuit 711 processes an image taken by the camera 707 and outputs as an HDMI (trade mark) signal.

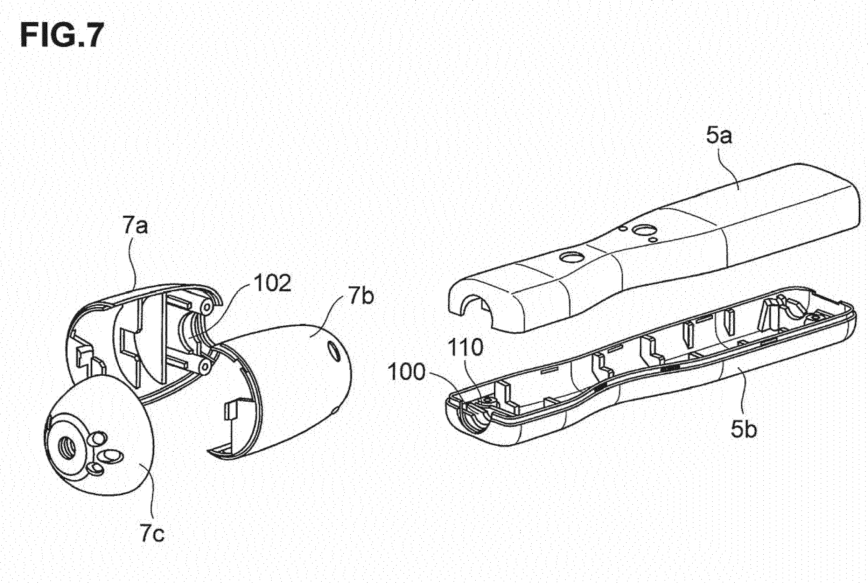

In FIG. 7, a chassis structure of the medical camera 2 is shown. The base part 5 is formed from an upper/lower body 5a and 5b. The apex part 7 is formed with left/right bodies 7a, 7b and a cap 7c.

Returning to FIG.6, since the flexible arm 9 is provided, a direction of the camera at the apex part 7 can be easily changed to a desired direction or position, and the camera can be easily held in position.

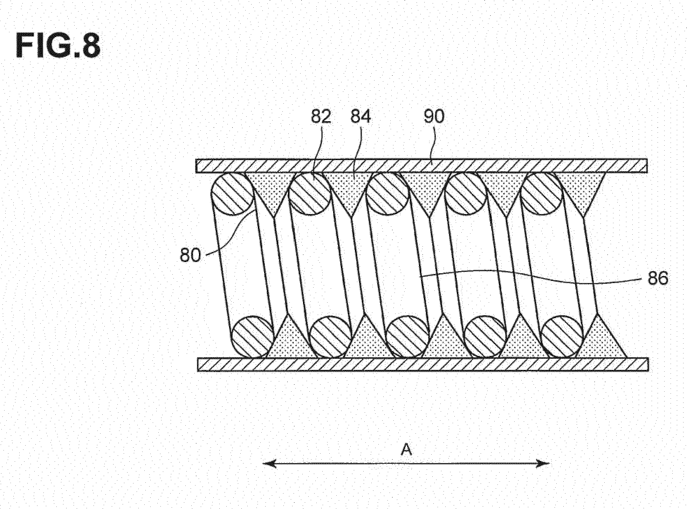

This flexible arm 9 is configured with the coil 80 and the tube 90 provided on an outer periphery thereof as shown in FIG. 8. In the tube 90, a wiring connecting the light source 705 and the control circuit 709, a wiring connecting the camera 707 and the image processing circuit 711 and the like are housed.

The coil 80 is formed as a double coil of a wire having a circular cross section (SUS304WPB) and a SUS wire having a triangular cross section (SUS304). In the present embodiment, a diameter of the wire 82 was set to 0.8 mm, and one side of the wire 84 was set to 1.0 mm Adjacent SUS wire (spring material) 82 and SUS wire (spring material) 84 are in contact with each other. In the SUS wire 84 having a triangular cross-section, one side of a triangle is provided so as to go along an outer periphery and a width becomes narrower with progression toward the inside (may be reversed). In a hollow part 86 of the coil 80, a cable 43 (for example, may be a thin coaxial cable) is inserted.

Along the outer periphery of the coil 80, a silicone tube 90 is provided so as to be in contact therewith. In the present embodiment, to an outer diameter of 54 mm of the coil 80, the tube 90 having an inner diameter of 50 mm in a natural state is used. Thus, by setting the outer diameter of the coil 80 slightly larger (from about 5% to 15% of a tube diameter) than an inner diameter of the tube 90, the tube 90 comes into close contact with the outer periphery of the coil 80.

Further, in the present embodiment, the tube 90 is brought into close contact with the coil 80 in a state elongated in an A direction of FIG. 8 from the natural state. Thus, since the flexible tube 90 tends to contract, a force may be applied in a compressing direction against the coil 80.

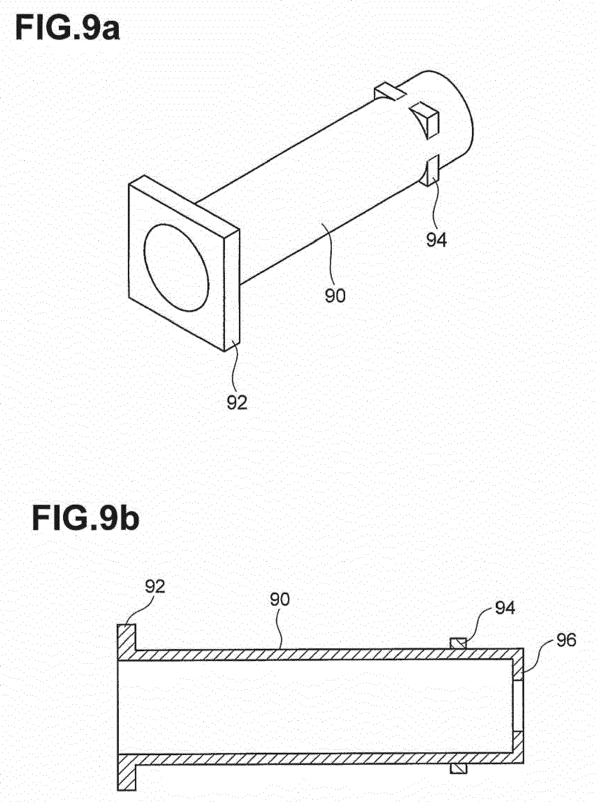

In FIG. 9a, a detail of the tube 90 is shown. In the present embodiment, the tube 90 is formed by molding flexible silicone. At one end, a flange 92 is provided. Also to the other end side, a flange 94 is provided.

In FIG. 9b, a cross sectional diagram of the tube 90 is shown. In an end part on a side on which the flange 94 is provided, a stopper part 96 which has a diameter smaller than a diameter of a hollow part is provided, and the coil 80 is prevented from falling.

As shown in FIG. 5, the lower body 5b of the base part 5 is provided with a concave part 100 for inserting the flange 92 of the tube 90. Though not shown in the drawing, also on a side of the upper body 5a, in the similar manner, a concave part is provided. Adjacent to the concave part 100, a stopper 110 is provided. The stopper 110 is provided to hold the coil 80 in a predetermined position when the flexible arm 9 is attached.

Further, also to the left/right bodies 7a, 7b of a movable part 7, a concave part 102 for inserting the flange 94 of the tube 90 is provided. Incidentally, the movable part 7 may not be provided with the stopper 110.

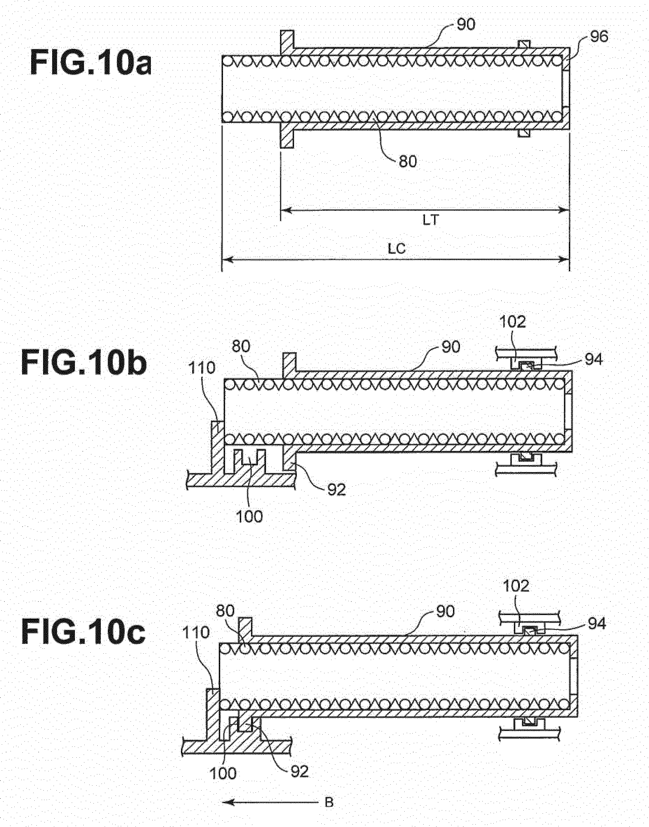

Next, a procedure of attaching the flexible arm 9 to the fixed part 5 and the base part 7 will be shown. First, as shown in FIG.10a, the coil 80 is housed in the tube 90. At this time, one end of the coil 80 is inserted until coming into contact with the stopper part 96 of the tube 90.

In the present embodiment, since a length LC of the coil 80 (for example 60 mm) is set longer (make longer by about 10% to 50% than a total length of the tube 90) than a length LT (for example 50 mm) of the tube 90, as shown in FIG. 10a, the coil 80 becomes a protruded state. As shown in FIG. 10b, when as in this state, the flange 94 of the tube 90 is filled in the concave part 102 of the movable part 7 and fixed, and the other end side of the coil 80 is brought into contact with the stopper 110 of the fixed part 5, the flange 92 of the tube 90 is not buried in the concave part 100 of the fixed part 5.

Therefore, as shown in FIG. 10c, after pulling the tube 90 in the direction of an arrow mark B, the flange 92 of the tube 90 is engaged in the concave part 100 of the fixed part 5. Then, a lid similarly provided with the concave part is topped.

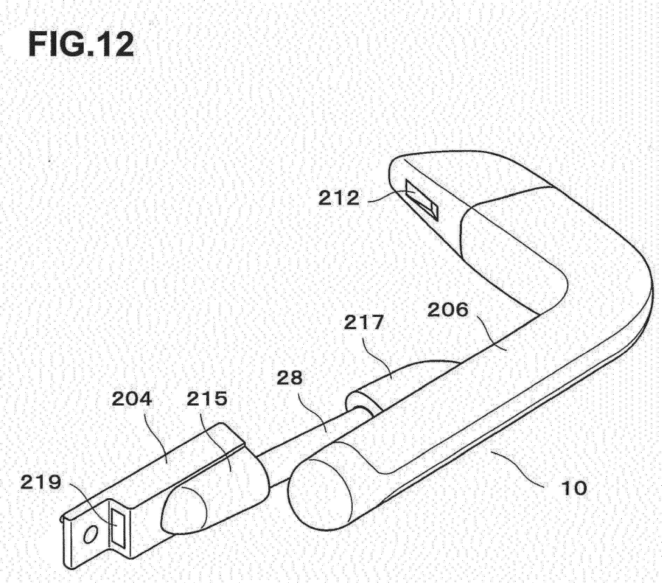

As shown in FIG. 3, on the right side, the HMD 10 is mounted. The HMD 10 is shown in FIG. 12.

This HMD 10 includes a fixed part 204, a movable part 217 and a flexible arm 208. The fixed part 204 is provided with an HDMI (trade mark) connector 219. The movable part 206 is provided with a prism 212 of a display. The movable part 206 covers the prism 212 in a light-shielding manner and has an opening only on a side that faces eyes of a wearer. Thus, by avoiding an influence of ambient light, a sharp image may be obtained.

At a center part (not end part) of the movable part 206, an arm-fixing part 217 is provided, and an apex part of the fixed part 204 is provided with an arm fixing part 215. A flexible arm 208 is provided between the arm fixing part 215 and the arm fixing part 217. When the flexible arm 208 is bent, a position or an angle of the prism 212 of the movable part 206 can be altered. Here, the flexible arm 208 has the same structure as the structure of the flexible arm 9 of the medical camera 2.

An HDMI (trade mark) cord 245 is connected to the HDMI (trade mark) connector 219 and lead to the first output terminal 16a of the switching device 6.

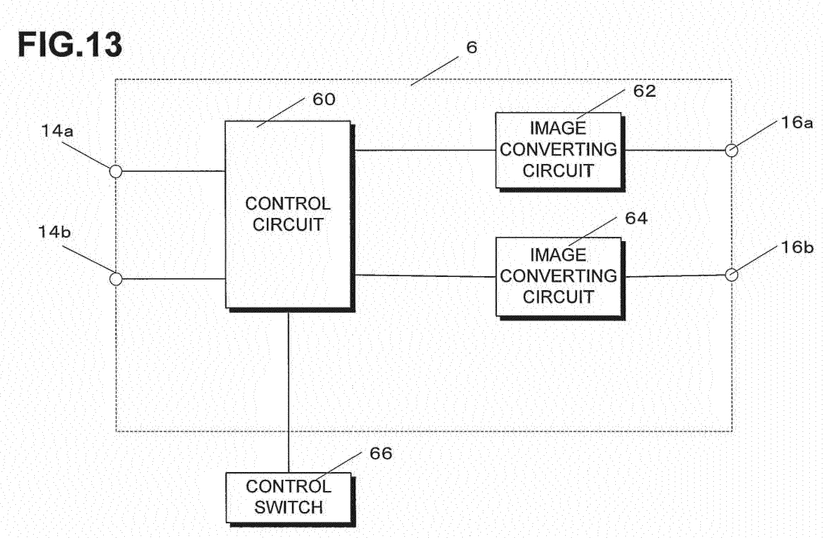

In FIG. 13, a circuit configuration of the switching device 6 is shown. The control circuit 60 switches whether the first input terminal 14a or the second input terminal 14b is connected to any one of the first output terminal 16a and the second output terminal 16b based on a signal from a control switch 66. Further, image-converting circuits 62 and 64 convert an image signal to a suitable image signal according to a device of an output destination. For example, since the HMD 10 is connected to the first output terminal 16a, the image converting circuit 62 converts the image signal so as to match it with its image size. Since the external monitor 12 is connected to the second output terminal 16b, the image converting circuit 64 converts the image signal so as to match it with its image size.

Returning to FIG. 2, the operator 600 operates a foot switch (control switch) 8 placed at the foot and switches an image displayed on the HMD 10 or an image displayed on the external monitor 12. Further, in this example, medical images such as SPECT images or the like are recorded in a PC 250 and are provided to the second input terminal 14b of the switching device 6.

The operator 600 may switch an image by only operating the foot switch 8. Further, by using the HMD 10 and the external monitor 12 together, a system in which the operator's sight line movement is only slight and other staff 610 and 612 are able to readily carry out a check by the external monitor 12 may be provided. The same image may be displayed on the HMD 10 and the external monitor 12. The switching device 6 may be configured so as to change an image provided to the HMD 10 in conjunction with an image provided to the external monitor 12.

### 3. Others

(1) In the above embodiment, a medical system was configured by using the medical camera 2. However, an intraoral camera 302 such as shown in FIG. 13 may be used. At an apex of a handle part 304, an apex part 306 is provided. In the apex part 306, a camera and a light source are housed. A circuit configuration is the same as that of FIG. 11.

For performing an imaging operation with this camera, the apex part 306 is provided with an imaging hole 316. Further, for performing illumination with a light source, the apex part 306 is provided with an illumination hole 318.

The camera is provided with a plurality of (6 pieces in the present embodiment) light sources (LEDs) corresponding to each of the illumination holes 318 corresponding to the imaging hole 316. In the present embodiment, a position of an apex of the illumination hole 318 is formed lower than a position of an apex of the imaging hole 316 (to be nearer to the handle 304). Thus, without the light from the light source directly coming into the camera, an appropriate imaging operation can be performed.

The handle 304 is provided with an illuminance changing switch 312. Every time when this switch 312 is pushed, an output of the light source is switched from a lower step to a higher step by a plurality of steps. After reaching a highest output, the output returns to a lowest output.

The intraoral camera 302 in the present embodiment is formed, as shown in FIG. 12, such that the apex part 306 has a predetermined angle (20□ in the present example) to the handle 304.

To the back end part of the handle 304, the HDMI (trade mark) cable (not shown in the drawing) is connected. This HDMI (trade mark) cable is connected to the first input terminal 14a of the switching device 6 for use.

According to the present example, the intraoral camera may be used for treatment of teeth in dental clinics.

In the above embodiment, every time the switch 12 or 312 is pushed, a light amount from the light source is changed. However, the light amount may be changed in accordance with a time in which the switch 12 or 312 is kept pushed.
(2) In the above embodiment, the foot switch 8 is used as the control switch. However, a switch that is manually operated may be used. Further in the above embodiment, switching between the output terminal 16a and the output terminal 16b is controlled based on a long push or a short push of the control switch 8. However, the control switch 8 may be provided independently to each of the output terminals 16a, 16b.
(3) In the above embodiment, the image converting circuits 62, 64 mainly change a size of an image. However, in addition to this, a form of an image may be changed by vertically flipping, rotating by 90°, mirroring of the input image.
(4) In the above embodiment, the switching device 6 switches the terminal that outputs the input image. However, in addition to this, a circuit for recording provided images in a portable recording medium may be provided. At this time, an image after conversion by the image converting circuits 62 and 64 may be recorded of course, but also an image before conversion may be recorded.

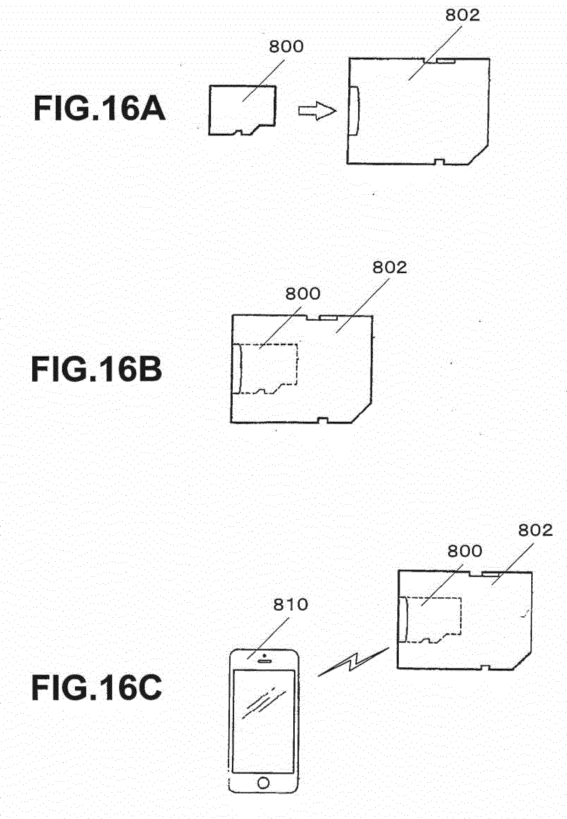

In FIG. 16, a method of recording data with a micro SD card 800 is shown. In this example, the micro SD card 800 is used by mounting on an SD card adapter 802 (see FIG. 16A). The SD card adapter 802 has the same external size as a full-size SD card. Therefore, when the micro SD card 800 is inserted into the card adapter 802, it may be used in the same way as a full-size SD card (FIG. 16B).

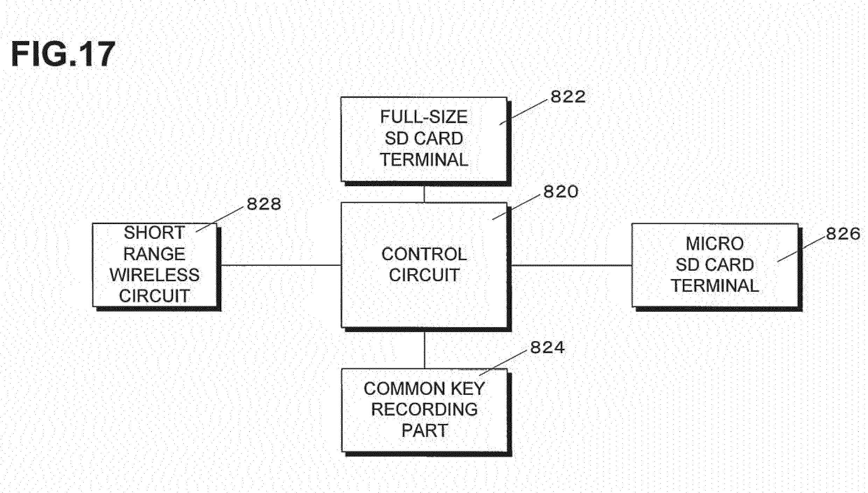

In the present example, since the switching device 6 is provided with a connector of the SD card, by mounting on this, image data may be recorded. In FIG. 17, a circuit block diagram of the SD card adapter 802 is shown. In the present example, a control circuit 820 (FPGA) for performing encipherment/decoding of the image data is provided.

Image data to be recoded are input from a full-size SD card terminal 822. The control circuit 820 enciphers the image data and outputs to a micro SD card terminal 826. Accordingly, the enciphered image data are recorded on the micro SD card 800.

Thus, the SD card adapter 802 with the micro SD card 800 in which enciphered image data are recorded is taken out of the switching device 6 and may be carried to other places. For example, when reproducing on the PC (not shown in the drawing), the following procedure is taken.

The SD card adapter 802 with the micro SD card 800 is inserted into an SD card slot of the PC. Then, by using a smartphone 810 (see FIG. 16C) and a short range wireless (Blue tooth (trade mark)), communication with a short range wireless circuit 828 (a circuit for Blue tooth (trade mark) communication) of the SD card adapter 802 is established. The control circuit 820 reads out a common key for decoding a secret code from a common key recording part 824 and transmits to the smartphone 810.

Then, by an operation of the user, the smartphone 810 (by treatment of application) transmits a complexed command and the common key to the SD card adapter 802. Upon receiving this, the control circuit 820 of the SD card adapter 802 decompresses the enciphered image data recorded on the micro SD card 800. Thus, on the micro SD card 800, un-enciphered image data are recorded. Therefore, this may be read and displayed by the PC or the like.
(5) In the present embodiment, since a double coil is formed such that a wire having a circular cross-section and a wire having a triangular cross-section are adjacent with each other, when the flexible arm 9 is bent, a degree of maintaining the bent state becomes higher. This is because a coil in which a circular wire and a triangular wire are adjacent with each other has larger return resistance than a coil in which the wire having a circular cross-section and the wire having a circular cross-section are adjacent with each other upon bending and may maintain a bent state. Further, since an inner diameter of the tube 90 is made smaller than an outer diameter of the coil 80 and, further, the tube 90 is brought into close contact with the coil 80 in an elongated state, the bent state may be maintained.
   Therefore, there is an effect that an angle or a position can be readily adjusted and the adjusted position can be maintained. Here the effect may be obtained by performing either one of, making the inner diameter of the tube 90 smaller than the outer diameter of the coil 80 (to make a force work in a radial direction) and bringing the tube 90 into close contact with the coil 80 in a state where the tube 90 is elongated (to make a force work in a direction in which an adjacent wire compresses).
(6) In the above embodiment, although the double coil is used as the coil 80, a single coil may be used. Also in this case, the bending maintaining effect due to the close contact/elongation of the tube 90 can be obtained.
   Further, not the double coil, but multi-coil more than three may be used. However, also in this case, it is preferable to wind a coil such that the circular wire and the triangular wire are next to each other.
(7) In the above embodiment, the coil 80 is formed with the circular wire and triangular wire. However, the coil 80 may be formed with a circular wire and a polygonal wire with four corners or more. Alternatively, not a polygonal but a rectangular (trapezoid, parallelogram and so on) wire may be used.
(8) In the above embodiment, the tube 90 applies a force in the direction of compression of the coil 80. However, energizing means that are fixed at both ends of the coil 80 and energized in the compressing direction (such as flexible member, spring or the like) may be provided.
(9) Further, in the above embodiment, the medical camera 2 is used by mounting on the eyeglasses 40. However, the medical camera 2 may be used by providing a clip or the like to the base body 5 and by fixing by fastening to a body or other objects.
(10) In the above embodiment, both the HMD 10 and the external monitor 12 display a simple image. However, an image obtained by combining a plurality of images may be displayed. For example, on the external monitor 12, two or more images such as vital data, an X-ray image, a MRI image, a medical camera image or the like may be displayed in combination. In this case, a combination of images to be simultaneously displayed may be changed by the control switch 66.
(11) In the above embodiment, the HMD 202 and the medical camera 2 are attached to the mounting fixture 20 by the guide member 14 and the attachment guide 30. However, a magnet may be used to attach these.
(12) Further, in the above embodiment, it is used as the medical camera and dental camera. However, it may be applied to cameras used in other purposes. For example, an industrial endoscope may be connected to the switching device 6.

## Claims

1. A medical system, comprising:
a medical camera that capture an image and outputs the camera image;
a medical image output device that outputs a medical image;
a head-mounted display mounted on a user;
an external monitor that the user or a person other than the user may see; and
a switching device having a selection control part, to which the camera image from the medical camera and medical data from a medical data output device are input, and which selects at least one of the camera image and the medical data to be shown in each of the head-mounted display and the external monitor.

2. A switching device, comprising:
a first input terminal for inputting a camera image from a medical camera that takes an image and outputs the camera image;
a second input terminal for inputting a medical image from a medical image output device that outputs the medical image;
a first output terminal for outputting an image signal to a head-mounted display mounted on a user;
a second output terminal for outputting the image signal to an external monitor that a user or a person other than the user can see; and
a selection control part, to which the camera image from the first input terminal and the medical image from the second input terminal are input, and which selects at least one of the camera image or the medical image to be shown in each of the first output terminal and the second output terminal.

3. The system according to claim 1 or the device according to claim 2, wherein the selection control part changes which image to be shown according to a signal from a control switch.

4. The system or the device according to claim 3, wherein the selection control part switches an image output to the first output terminal when an on-signal from the control switch is continuously input for a predetermined time period or shorter and switches an image that is output to the second output terminal when the on-signal from the control switch is continuously input exceeding the predetermined time period.

5. The system or the device according to any one of claims 1 to 4, wherein the control switch is a foot switch.

6. The system or the device according to any one of claims 1 to 5, wherein the selection control part at least flips vertically, rotates by 90□, or mirrors an image, which is output to the first output terminal or the second output terminal or to both upon receiving a control signal.

7. The system or the device according to any one of claims 1 to 6, wherein the selection control part includes recording means for recording an image from the first input terminal, the second input terminal, or both of these on a portable recording medium.

8. The system or device according to any one of claims 1 to 7, wherein the medical camera is mounted on a body of the user.

9. The system or device according to any one of claims 1 to 7, wherein the medical camera is an intraoral camera.
